# EUROPEAN PATENT APPLICATION

(11) **EP 2 172 231 A1**
(43) Date of publication of application: **07.04.2010**
(21) Application number: 08715302.9
(22) Date of filing: 24.03.2008
(51) Int. Cl.: A61L 27/38, A61L 27/40, A61F 2/08, A61K 35/12, C12N 5/00, C12N 5/06

(54) **TISSUE ENGINEERING TENDON AND CONSTRUCTION METHODS IN VITRO THEREOF**

(30) Priority: 29.06.2007 CN 200710043013
(71) Applicant: Shanghai Tissue Engineering Life Science Co. Ltd., Shanghai 200235 (CN); Shanghai 9th People's Hospital, Shanghai Jiaotong University School Of Medicine, Shanghai 200011 (CN)
(72) Inventor: CAO, Yilin, Shanghai 200011 (CN); LIU, Wei, Shanghai 200011 (CN); XU, Feng, Shanghai 200011 (CN); DENG, Dan, Shanghai 200011 (CN); LI, Hong, Shanghai 200235 (CN)
(74) Representative: WSL Patentanwälte
(86) International application number: PCT/CN2008/070566
(87) International publication number: WO 2009/003375

(57) **Abstract**

The invention discloses a tissue engineering graft, comprising:(a) pharmaceutically-acceptable biodegradable material; and (b) seed cells, which can be inoculated on the described biodegradable material and are selected from:(i) fibroblasts; (ii) adipose derived cells, or (iii) mixture of dermal fibroblasts and ASCs according to the ratio of 1:10000-10000:1. The graft can be prepared by mixing of seed cell and pharmaceutically-acceptable biodegradable material, obtaining a construct of seed cells and pharmaceutically-acceptable biodegradable material, then culturing the construct in a bioreactor in vitro. The graft can be used for repairing the defect of tendon tissues.

## Description

This invention generally relates to medicine and biomedical engineering field, especially to the construction methods of in vitro tendon tissue engineering using fibroblasts and/or adipose-derived cells as well as the use thereof.

### BACKGROUND

Tendons, parts of skeletal muscles, are dense collagen connective tissue bundles which attach muscles and bones. Tendons can be considered as remains or expended parts of muscles. The fresh samples of tendons are silvery white, elongated, tough, yet somewhat pliable. Tendon is arranged in aligned bundles with a characteristic wave-like, or crimp pattern. While an external exceed 4% stress was loaded on the tendon, this waviness, called crimp, would first straighten out prior to any elastic or plastic deformation of collagen. Conversely, the crimp structure appears when tendon goes back in loose condition. Collagenous fibers penetrate into the endomysium, attach to muscle fibers' sarolemma in muscle-tendon joint, while in tendon-bone joint, tendon bundles attach to bones directly, where most of collagenous fibers grow into bones, forming Sharpey's fibers. Spanning between skeletal muscle and bone, tendon transmits muscle force to bone attachments to effect movement. Tendon sustains great stress but has no contract ability.

Tendon defects are among the most common orthopaedic injuries experienced by patients. Three main options have been utilized for repair or replacement of a damaged tendon: (1) autografts, (2) allografts, (3) synthetic prosthesis. However, these options above all have some drawbacks. For example, (1) autograft has to face a limited tissue resource from patient; (2) tendon transplant using fresh allograft might lead to serious immune rejection, while freeze-dried allograft has to be replaced by autologous tendon cells in the end, as freeze treated allograft only has collagenous fibers left inside; then (3) although synthetic tendon replacement could get a strong mechanical force in a short term, it may degrade in a period of long time, and lead to inflammation, fibrosis and tendon adhesion reaction, even exclude grafts outside patient's body.

Tissue engineering, emerged and flourished at the late 1980s, makes all those difficulties resolvable. Yilin Cao firstly reported reconstruction of tissue-engineered tendon-like tissue in nude mice successfully in 1994, using a scaffold material of polyglycolic acid (PGA). Then Yong-tao Liu successfully reconstructed tissue-engineering tendon in hen, an immuno-competent animal, by replacing the animal's second digital flexor profundus tendon with autologous tendon cell. However, new injuries might be made as a large amount of tendon tissues have to be used, by utilizing autologous tendon cells as seed cells in tendon tissue engineering. Therefore, seeking a new source of seed cells is the chief problem in the development of tendon tissue engineering.

In addition, recent global research about tendon tissue engineering is mainly focusing on *in vivo* repair experiments on animal's model, that is to say, a large amount of seed cells are firstly amplified *in vitro* and seeded onto bio-materials, then those cells-scaffold complexes are finally transplanted into defect areas *in vivo* as soon as possible, or after a short-term culture for only one or two weeks. Although some primary success is achieved, this method, constructing tendons by directly transplanting cells-seeded scaffold *in vivo,* is found to have some basic withdraws, including (1) bad growth condition of seed cells in scaffold leads to transplant failures, or low unsustainable success rate, as transplant stuff are cells-scaffold complexes, not tendon grafts; (2) acid scaffold degradation materials lead to inflammation, scar formation and adhesion of tendon, therefore affect repair effectiveness directly; (3) injuries in high tension areas could not be cured, as bio-force of cells-biomaterial composition decreases rapidly when scaffold degrades. In conclusion, clinic usage could not be recently realized as difficulties above all still existed, although a lot of tendon reconstruction and repair experiments were conducted in animals' bodies.

Therefore, obtaining large number of functional tendon cell is the most crucial problem, not only in tissue engineered tendon reconstruction and injured tendon repair, but also in mass process of tissue engineering tendons. As tendon cell could lose their ability of secreting cell matrix in the process of cell passage, it is rather difficult to get enough functional tendon cells to repair a large area of injured tendon by tendon tissue engineering. The second crucial problem is how to get a relatively matured tissue engineered tendon with the ability of high tension tolerance, as well as most of its scaffold being break down when in use. In sum, searching a broader source of seed cells and finding a more optimized reconstructive technique are the central issues in tendon tissue engineering research.

To resolve the first main problem, several researchers tried hard to find new sources of seed cells, and make effort to stimulate amplification of the cells.

The first resolution is to find new replaceable cells, such as mesenchymal stem cell (MSC) and dermal fibroblast (DF). MSC, with a capacity of multidirectional differentiation, is a kind of cells that could differentiate into different sorts of mesenchymal cells in some specific induced condition. Awad found MSC-collagen compositions, which were transplanted in artificial patella ligament injuries, could form a preliminary engineered tendon with stronger mechanical property than that of control group (collagen group), although there was no obvious differences in histological and morphological observations. However, MSCs are difficult to be isolated, and have a rather limited cell source. Chen Bing proved that DFs was a possible cell source in tendon tissue engineering and ligament reconstruction. In whose successful experiments, pig's autologous DFs were used to reconstruct tissue engineered tendons, which were implanted into the animal's injured flexor digital superficial tendon.

The second resolution is to accelerate cell amplification and stimulate cell matrix synthesis. Growth factors, affect cell function by cell signal transduction, are a kind of polypeptide factors that could stimulate or inhibit cell amplification *in vivo* or *in vitro.* Several scholars study the positive effects of growth factors in injured tendon repairing by directly adding or blocking those growth factors, while other researchers use gene transfer technique to change cellular function. The central idea of this technique is adjusting cell growth to tissue repair progress by stimulating or inhibiting protein synthesis and secretion in gene-transfer targeted cells. Using this technique, cells and tissues could be regulated to grow as will, as synthesis and secretion of cytokines could be well controlled. Some scholars have successfully transferred genes to tendons in different strategies, and some data suggested that transferred lacZ report gene could be expressed constantly for 6 weeks in patella ligament. In Lou's experiment, lacZ gene was transferred into chicken flexor digital tendon by recombinant adenovirus. Results showed that the transferred lacZ gene remained stable for 75 days in the tendon and tendon sheath. The author also pointed out some ways to improve healing and avoid adhesion.

However, the problem of lacking enough tendon cell source is still not completely solved, in spite of the successful researches above.

To resolve the second main problem, several kinds of biomaterials have been tried on tendon tissue reconstruction. De-jun Cao got a tendon-like structure by using polyglycolic acid (PGA), a kind of absorbable biomaterial, and tendon cell. In whose experiment, the tendon cell seeded PGA scaffold changed to a kind of tissue with characteristics similar to normal tendon, after 6 weeks' *in vitro* culture on U-shaped spring, which gave the cells-biomaterial composite a sustainable tension. The resulted tendon-like tissue could resist to some degree of external loaded force. However, quantity, frequency and other tension parameters loaded by spring cannot be exactly measured in this method, as well as low stress resistant in this kind of engineering tissue. Therefore, *in vitro* culture environment, a simulation to *in vivo* niches, should be future improved and optimized.

In conclusion, a new kind of seed cell should be used in human tendon tissue engineering *in vitro,* and the reconstructed tendon should obtain good mechanical properties.

### CONTENT OF THE INVENTION

One object of the invention is to provide a kind of tissue engineered human tendon graft.

Another object of the invention is to provide an *in vitro* method of preparing the above said tissue engineered tendon graft. Another object of the invention is to provide a use of the above said tissue engineered tendon graft.

In the first aspect of the invention is to provide a tissue engineered human tendon graft comprising:
(a) pharmaceutically acceptable biodegradable material; and
(b) seed cell, which can be inoculated on the described biodegradable material and is selected from: (i) fibroblast; (ii) adipose derived cell, or (iii) mixture of fibroblast and adipose derived cell according to the ratio of 1:10000-10000:1.

In another preferred example, the tissue engineered tendon graft has a max tension of 10-80N.

In another preferred example, the seed cell is a fibroblast or the mixture of fibroblast and adipose-derived cell.

In another preferred example, the content of said seed cell is from 1×10⁵ cell/ml to 5×10⁸ cell/ml.

In another preferred example, said biodegradable material is in funicular shape, net shape (such as peri-string bag), sheet shape (such as used for surrounding ectoblast), liquid (such as collagen or chitosan solution), or the complex scaffold formed by the materials of the above mentioned shape.

In another preferred example, said fibroblast and adipose-derived cell is an autograft or allograft.

In another preferred example, the pharmaceutically acceptable biodegradable material is selected from the group consisting of polylactic acid, polyglycolic acid, polyhydroxybutyric acid, polyanhydride, polyphosphazene, polyamino acid, pesudo-polyamino acid, polyorthoester, polyesterurethane, polycarbonate, polyethylene glycol, polyethylene oxide, poly-dioxanone, collagen, gelatin, glycosaminoglycan, chitosan, chitin, alginate, calcium alginate gel, acellular matrix, polycaprolactone, and their mixtures.

In the second aspect of the invention providing a method of preparing the above said tissue engineered tendon graft, comprising the steps of:

Mixing the seed cell with the pharmaceutically-acceptable biodegradable material, obtaining a seed cell-biomaterial composition, wherein the seed cell is inoculated on the biodegradable material and is selected from:(i) fibroblast; (ii) adipose derived cell, or (iii) mixture of dermal fibroblast and adipose derived cell according to the ratio of 1:10000-10000:1.

In another preferred example, further comprising the steps of:

Culturing the seed cell-biomaterial composition in bioreactor to obtain the above said tissue engineered tendon graft.

In another preferred example, said bioreactor is a pull-draw tendon apparatus disclosed in the Application No. 200510110037.0 and the improved series bioreactor based on it.

In another preferred example, the content of the seed cell in the graft is from 1×10⁵ cell/ml to 5×10⁸ cell/ml.

In another preferred example, the external tension loaded on the seed cell-biomaterial composite in the bioreactor is from 2 N to 20 N.

In another preferred example, the biodegradable material is in funicular shape.

In the third aspect of the invention providing the use of the above said tissue engineered tendon graft in the manufacture of a graft for repairing the damage of tendon.

Therefore, the invention provides a tissue engineered human tendon that using a novel seed cell and constructed *in vitro.* Moreover, the described tissue engineered tendons have good mechanical properties.

### BRIEF DESCRIPTION OF THE DRAWING

FIG. 1 is a picture of the 2nd passage human fibroblasts.
FIG. 2 represents the 2nd passage tendon cell.
FIG. 3 represents the primary adipose derived cells.
FIG. 4 represents the identification about different phenotypes of adipose derived cells, wherein
Fig 4A represents Vimentin⁺; Fig 4B represents CD106⁺; Fig 4C represents CD34⁻; Fig 4D represents CD29⁺/CD49D⁺; Fig 4E represents CD44⁺/CD49D⁺.

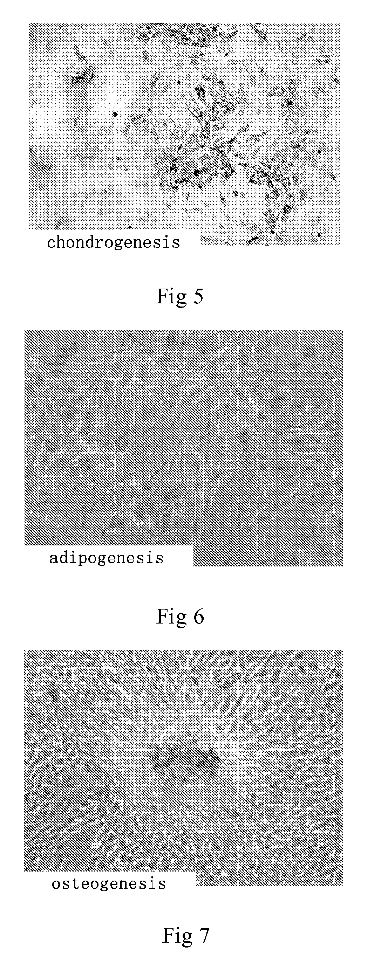

FIG. 5 represents type II collagen expression pattern of the adipose derived cells being induced to chondrocytes.
FIG. 6 represents the lipid droplets formation in adipogenic differentiated cells' cytoplasm, the lipid droplets have been stained by oil red staining.
FIG. 7 represents the formation of calcium nodules in osteogenesis induced adipose-derived cells.

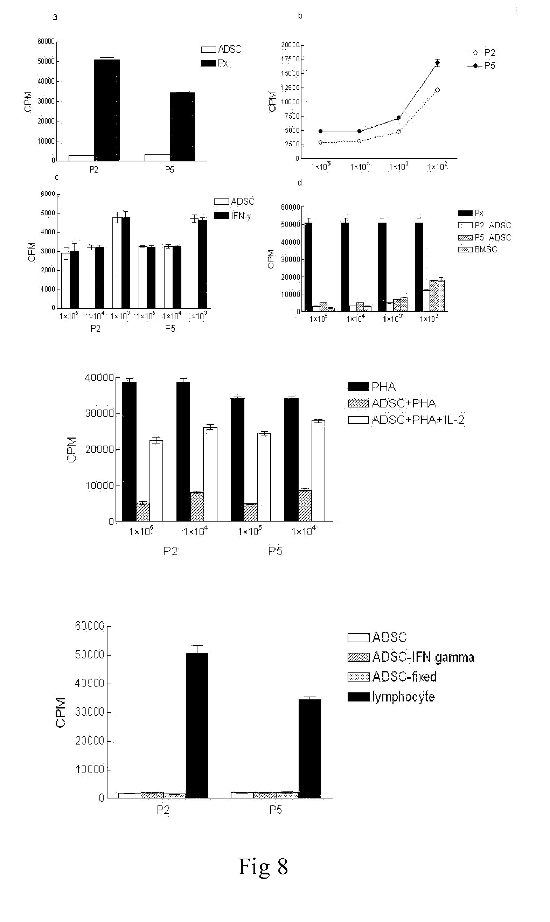

Figure 8 represents the immunoregulation result of adipose derived cell.
Figure 9 represents the fascicular PGA scaffold after pre-moulding.
Figure 10 represents the pre-moulded bundle PGA fiber scaffold mounting on a U-shape spring.
Figure 11 represents the appearance ofbioreactor.
Figure 12 represents the construction method of in vitro tendon engineering in a bioreactor with human dermal fibroblast and/or adipose derived cell and PGA scaffold.

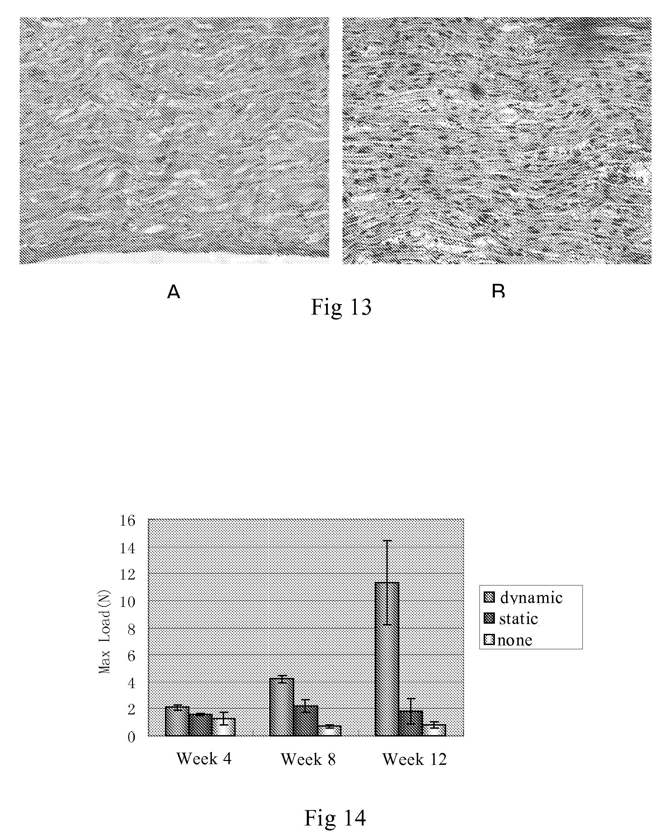

Figure 13 represents the histology examination result of tissue engineered tendon after static stretch for 2 weeks plus dynamic stretch for 10-15 weeks using human dermal fibroblast as seed cell, wherein, A represents for skin fibroblast+PGA; B represents for tendon cell+PGA.
Figure 14 represents the biomechanical properties of in vitro tissue engineered tendons constructed under different tension.

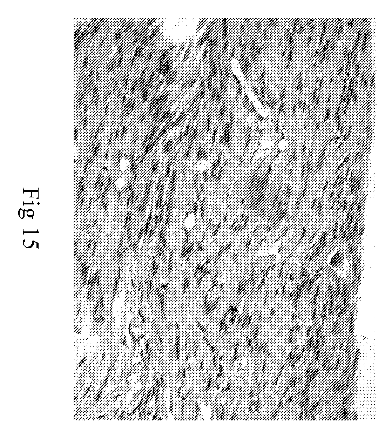

Figure 15 represents the histology examination result of tissue engineered tendon after static stretch for 2 weeks plus dynamic stretch for 10-15 weeks using human adipose derived cell as seed cell and PGA fibers as scaffold.

### EXAMPLES

Under extensive and in-depth research, the inventors were surprised to find that human skin fibroblasts, adipose derived stromal/stem cells (ASCs) or their mixture could be used as seed cells for tissue engineered tendon reconstruction. Moreover, tissue engineered tendon with good mechanical property could be obtained by incubating the cells-scaffold component in bioreactor *in vitro.*

### TERMS

The term "purified or isolated" means the purified or isolated material containing no other cell, protein or polypeptide.

The term "xenograft" refers to the method transplanting a required biological material (e.g. tendon) obtained from one species to another species.

The term "autograft" refers to the method transplanting a required biological material (e.g. tendon) removed from a patient to the same person.

The term "allograft" refers to the method transplanting a required biological material (e.g. tendon) removed from an individual to another patient in the same species.

As used in this patent, "pharmaceutical acceptable biodegradable material", "medically acceptable biodegradable material", "biodegradable material", "biomaterial" and "material" are the same thing, means the biodegradable material that serve as a scaffold for cells and growth factors. The described materials have a good biocompatibility with cells and tissues and a degrade rate matching to that of tissue growth.

As used in this patent, "tissue engineered graft", "tissue engineered tendon graft" and "tissue engineered tendon" can be exchanged, as long as all of those terms describe a cells-scaffold graft that could be used to repair tendon defect *in vivo*.

### SEED CELLS

Seed cells used in this invention are fibroblasts and adipose derived cells coming from either autologous or allogeneic individuals, though autologous fibroblasts are better than allogenous fibroblasts. When the seed cells come from mixtures of fibroblasts and adipose derived cells, the available proportion is 1:10000-10000:1, 1:5-100:1 is better, and 1:2-10:1 is the best choice.

Have no specific restriction in cell source, the fibroblasts in this invention could be gathered from any source. Usually the fibroblasts in this invention are autologous (e.g. fibroblasts from dermis, subcutaneous tissues, and other tissues) or allogenous (e.g. human foreskin fibroblasts) fibroblasts. Other cells of adipose stem cells, bone marrow stromal cells, or other kinds of stem cells also could be used.

Human fibroblasts are used in this invention.

Although the autologous fibroblasts are preferred, allogeneic fibroblasts could also be used.

In another preferred example, the seed cells are human autologous dermal fibroblasts.

The isolation method of dermal fibroblasts is the method widely used in this field. The common used isolation methods are:
(a) Collagenase isolation: briefly, dermis harvested under sterile condition is minced into small pieces (2×2×2mm³). Then the tissue fragments are rinsed with phosphate-buffered saline (PBS, containing 100 U/ml penicillin, 100 U/ml streptomycin) for two times followed by the digestion with twice the volume of 1 mg/ml type II collagenase (Worthington, Freehold, NJ, USA) in serum-free Dulbecco's modified Eagle's medium (DMEM, Gibco, Grand Island, NY) at 37°C on a rotator. The resulting cell suspension harvested at 4h post-digestion is filtered through a sterile nylon mesh to remove tissue residues. The cells in precipitation are counted after washed twice in PB S, then the cell viability of dermal fibroblasts is tested by Trypan Blue staining. The extracted cells are plated on 100 mm culture dishes (1×10⁶cell/dish) and incubated at in a humidified atmosphere containing 95% air and 5% carbon dioxide.
(b) Tissue culture: dermis harvested under sterile condition is minced into small pieces (2×2×2mm³). Then the tissue fragments are rinsed with phosphate-buffered saline (PBS, containing 100 U/ml penicillin, 100 U/ml streptomycin) for two times. Tissue fragments are put on dish surface evenly followed by incubation at 37°C for 2-4 hours. Tissue fragments are immerged by DMEM carefully so that cells could grow out of the tissue fragments.

The culture method of dermal fibroblasts is also the method widely used in this field, so is the culture median. One preferred method is to incubate dermal fibroblasts under a humidified condition containing 95% air and 5% carbon dioxide. Suitable culture media include but not limited to: 1) DMEM(Gibco) +10% fetal bovine serum; 2) DMEM+20% fetal calf serum; 3) DMEM+10-20 % autologous (allogeneic) human serum. In addition, a variety of growth factors (e.g. cytokines promoting fibroblast growth), genetically modified ingredients or cell components are added to the described culture medians.

Dermal fibroblasts suit for this invention should have the ability to amplify *in vivo* or *in vitro.* One preferred type of dermal fibroblasts is the cells cultured *in vitro* below passage 50. The expression of type I collagen is proved by immunohistochemistry, and the mRNA expression of I type collagen is proved by in situ hybridization or RT-PCR.

The inventors discovered that the adipose derived cells acquired in the adipose tissue also belong to a kind of MSC, similar to BMSC, they have muti-lineage differentiation potentials and can be induced to differentiate into bone, cartilage and tendon cells. ASCs still have other advantages over BMSCs as follows: (1) The source of ASCs is more abundant and easier to obtain, and the quantity is much higher than that of BMSCs, and the cell proliferating capability is also much better than that of BMSC, and thus can provide ample amount of cells for tissue engineering. (2) The ability of producing collagen is stronger. Because tendon tissue needs a great deal of type I collagen to maintain enough tensile strength, ASCs are more suitable as the tendon seed cells, and more likely to form the functional tendon tissue. (3) With low immunity and immune modulation function, ASCs are more suitable for allograft implantation and have more extensive clinical application potentials.

Therefore, adipose derived stromal/stem cells (ASCs) not only can be used as tissue engineered tendon seed cells, and but also have very high practical value, developing the related research will probably form the tissue engineered tendon that could be accepted by different individual patients who need tendon graft treatment and thus to break the bottleneck problem of individual based engineered tendon treatment.

The method of separating and acquiring adipose derived stromal/stem cells are what have been already known in the skill area. One preferred method is:

After liposuction surgical procedure, the abandoned human adipose tissue can be transferred to a cell culture bottle, washed with normal saline followed by the treatment of 0.075% type I collagenase (Worthington, Freehold, NJ, USA) in the same volume on the shakers at 37 □for digestion for an hour, and then centrifuged under 300 g for 10 minutes to get cell pellets, After removing the supernatant as well as the fat tissue, the cell pellets were resuspended in culture medium and seeded at the density of 1 × 10⁶/dish (the 10 cm culture dish).

The culture method and culture medium for adipose derived stromal/stem cells are also well established in this area. The method of culturing ASCs includes the placement into an incubator with the saturated degree of humidity, 5% CO₂ .The suitable culture medium includes: (but is more unlimited than) 1) DMEM(Gibco) +10% fetal bovine serum; 2) DMEM+20% fetal calf serum; 3) DMEM+10-20 % autologous (allogeneic) human serum. Add various growth factors (for example promoting the ASCs growth etc.), various compositions of gene transfection reagents, and various cell components. The ASCs that are applicable to the invention should have the ability to grow outside and inside human body. One optimized method of the culture is to be able to culture ASCs up to 30 passages in vitro from primary culture. If necessary, tendon cells or bone marrow stromal cells can be added into the seed cells culture. The method to harvest and isolate tendon cells has been already well known in the skill area. One preferred method is to digest tendon tissue with collagenase, i.e. diluting the collagenase to the concentration of 0.1-5mg/ml (prefer 1mg/ml) in DMEM (Gibco) and digesting tendon tissue in this solution at trypsin 37 □ ±2 □ on a rocker for about 4-20 hs.

Tendon cell culture method and culture medium are also well known in the skill areas. One preferred method is to culture tendon cells in an incubator with saturated degree of humidity and 5% CO₂. The suitable culture medium includes:(but is more unlimited than) 1) DMEM(Gibco) +10% fetal bovine serum; 2) DMEM+20% fetal calf serum; 3) DMEM+10-20 % autologous (allogeneic) human serum. Adding various growth factors (for example growth factor promoting tendon cell growth), various gene transfection reagents, various cells in the above-mentioned culture medium

The source of bone marrow stromal cells (BMSC) didn't specially limit in the invention, one preferred source is the marrow that comes from the autologous. The method of harvesting and isolation of bone marrow stromal cells is what have been already known in the skill area. One preferred method is harvest bone marrow under anaesthesia and isolate nucleate cells with gradient centrifugation, and culture the cells in suitable medium of DMEM (Gibco, Gland Island, NY, USA) containing 10% fetal bovine serum, 300mg/mLofL-glutamine, 50mg/mLof vitamin C , 100U/mL of penicillin, and 100U/mL of streptomycin and 5nM of dexamethasone (Sigma). The cells are cultured at the density of about 1 × 10⁵/cm² in culture medium at 37 °C with 5% CO₂ and 100% saturated degree of humidity. Medium can be changed at the first 48 hours, and conditional medium can be added to continue the culture until next medium change. When the cells reach confluncy, they can be treated with 0.25% trypsin + 0.02% EDTA for cell detachment and further passaged at the density of 1 × 10⁴/cm².

### Biodegradable materials

The materials used in the present tissue engineered tendon reconstruction are biodegradable materials, including but not limited to
(a) degradable synthetic macromolecule material, such as poly α-hydroxy acids(polylactic acid (PLA), polyglycolic acid (PGA) and polyhydroxybutyric acid (PHB), etc.), polyanhydrides, polyphosphazenes, polyamino acid, pesudo-polyamino acid, polyorthoesters, polyesterurethane, polycarbonate, polyethyleneglycol , poly (1,4-dioxan-2-one), polyethylene oxide and polydioxanone, etc.;
(b) natural degradable material, such as collagen, gelatin, glycosaminoglycan (GAGs), chitosan, chitin and alginate, and calcium alginate gel.etc.;
(c) the mixture or composition of above-mentioned material, especially the composition of macromolecule material and natural material.

Among them, PGA and PLA, or PLGA etc are preferred.

### Bioreactor

The bioreactor that is applicable to the invention didn't specially limit, can use various in common use bioreactors of the invention. A preferred bioreactor has the following characteristics: (1) The size, capacity is suitable, therefore, the metabolism waste can be easily removed, and it should not be contaminated. (2) It can mimic the in vivo niche of tendon microenvironment like the proper biomechanical loading, and therefore to provide an environment fitting for tendon tissue development in the bioreactor. (3) Parameters such as mechanical loading force and frequency are controllable and easy to operate

### Tissue Engineered Tendon

The invention provided a kind of new tissue engineered tendon graft comprising :(a) the pharmaceutically-acceptable biodegradable material; and (b) the dermal fibroblast and/or adipose derived cells, the two seed cells can be either individually or combinationally seeded onto the biodegradable materials. The tissue engineered tendon provided by this invention possesses good mechanical property, and can be tested using the routine methods normally used in this area, for example but is more unlimited instruments such as the application Intron biomechanical measurement instrument, BOSE biomechanical measurement instrument to test the tensile strength of engineered tendon grafts. The invented tissue engineered tendons may have the optimal maximal loading force of 10-80 Newtons, and the best loading force about 40-80 Newtons.

The tissue engineered tendon of the invention is relatively mature, after in vitro culture for a certain time period, the scaffold materials are mostly degraded and thus to avoid tendon adhesion and breading caused by acidic degradation product of the scaffold materials, it is thus designed to facilitate proper repair of tendon defect using the invented tissue engineered tendons.

The tissue engineered tendon of the invention is relatively mature, after in vitro culture for a certain time period, the seed cell secreted sufficient extracellular matrix such as collagen, and made the newly generated tissue engineered tendon already having a certain level of mechanical strength and thus to avoid the breaking of the implanted tissue engineered tendon, etc. The shape of the invented tissue engineered graft does not have special limits, can be any shape to fit the requirement of tendon repair, but usually a long cord form.

The in vitro cultured and expanded seed cells will form a cell-scaffold construct after being seeded on biocompatible and biodegradable scaffold materials. After culturing inside a bioreactor for a certain time period, the cell-scaffold will form a relatively mature tissue after the degradation of scaffold materials and seed cell proliferation and matrix production to replace the degraded scaffold materials.

The bioreactors commonly used in this area can be used for this invention, as long as it can provide the necessary dynamic mechanical loading. A preferred model will be the one that can provide a stretching mechanical stimulation. To proceed, the size and the length of the graft will be decided and then the cell-scaffold construct will be cultured under the condition of mechanical loading (2-20 Newtons) in vitro for a week with medium change every one or two days to maintain the cell nutrition supply and thus form a transplantable tendon graft.

The concentration of the seed cell in the present tissue engineered tendon is usually about 1×10⁵/ml to 5×10⁸/ml, preferably 1×10⁶/ml to 1×10⁸/ml, more preferably 5×10⁶/ml to 5×10⁷/ml. The seed cell concentration is usually adjusted with culture medium volume, then were mixed with degradable material. There will be no special limit in the ratio of the cell-containing solution volume to the volume of scaffold, but a maximum volume that the scaffold can absorbed will be preferred. In addition, other cell components, growth factor or various gene transfer reagents can also be added in this invented tissue engineered tend graft to keep the tendon cell phenotype, promote tendon cell growth, and promote the neovascularization of the tissue engineered tendon after in vivo implantation.

The tissue engineered tendon graft or tendon by this invention can be directly transplanted at the defect of human body to repair tendon defects.

The above-mentioned characteristic that the invention speaks of, or Example the characteristic spoken of can arbitrarily combine. All characteristics have been traced by this case manual can counteract with any combination thing form, in the manual each characteristic announced to public, can be replaced by any can provide a homology, equalization or the acting for of alike purpose vicarism characteristic. Consequently in addition to specially explaining, the characteristic have been traced only is the general example of equalization or alike characteristic. The main advantages of the invention are:
(1)The dermal fibroblast and (or) adipose source cell are widely distributed in human body and thus are a easy source for cell harvest and can solve the problem of seed cell source shortage for tendon engineering.
(2)The adipose derived stem cells have low immunity and immune modulation function and thus may help to solve the problem of immune rejection of allograft transplantation.
(3)Can be reshaped according to the shape of the defect tissue and the engineered tissue can survive life long after in vivo implantation.
(4) With the use of new bioreactor, a precise and controllable uniaxial mechanical stretch can be provided and excluded volume effect (EVE) effect by large molecules can be applied to accelerate the maturity of tendon.
(5)After the formation of in vitro engineered tendon, cells will be wrapped with the natural extracellular matrix to help maintain the cell survival after in vivo implantation and thus to obtain a relatively higher success rate of graft transplantation.
(6) By in vitro engineering, the scaffold material of the prepared graft can be mostly degraded during in vitro culture before transplantation, and implanted tendon graft will not produce acidic degradation products and thus to avoid fibrotic reaction and adhesion to implanted tendon graft.
(7)The in vitro engineered tendon can have a certain level of mechanical strength and thus allow for functional exercise like the natural tendon graft to prevent graft fibrotic adhesion and promote functional recovery.

In the followings, a concrete example is used to elaborate the invention further. Should comprehend, this example is only used for explaining the invention but not for limiting the application area. For the experimental methods in the following Example, if the concrete condition is not noted, usually it should be carried out according to normal regulations condition or according to the condition suggested by manufacturer. Unless moreover explain, otherwise all percentage and number press the weight to account.

Unless define separately, in the text use of all profession and science terminology are the same with the one commonly used by skill area well-trained personnels. In addition, any similar or equipotent to the contents of method and material all can be applied to the invention method in. In the text the preferred implementation method and material are only used for example demonstration.

### Example 1

The isolation and culture of skin fibroblast

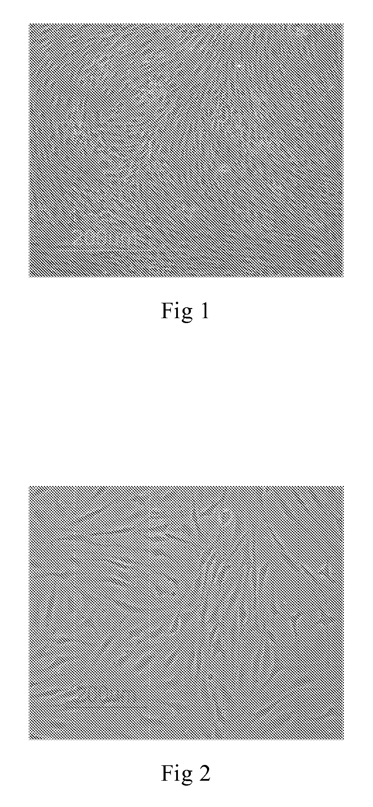

The abandoned foreskin tissue is taken during an operation under sterile condition, cut into 2×2 mm³ size tissue fragments, washed with phosphate buffered saline (PBS, containing 100 U/ml penicillin, 100 U/ml streptomycin) for 2 times, added with double volume of 1 mg/ml type II collagenase (Worthington, Freehold, NJ, USA) and placed in shakers to digest 37 □ for 4 h, then the digested cell solution was filtered through a sterile nylon mesh followed by centrifugation, the precipitated cells were washed with PBS for two times and counted, and Trypan blue stained to detect the vitality of the fibroblasts, the cells were then seeded at the density of 1×10⁶/dish (100mm culture dish), cultured in DMEM (Gibco, Gland, Island, NY, USA, containing 10% fetal bovine serum, 300 ug/ml L-glutamine, 50 ug/ml vitamin C, 100 U/ml penicillin, 100 U/ml streptomycin), fibroblasts are passaged to the next generation, expanded and digested with 0.25% trypsin to collect before the use in the experiment (Figure 1).

### Example 2

The isolation and culture of tendon cells

The fresh abandoned human tendon tissue is obtained from amputated leg after the operation under sterile condition, cut into 2×2×2 mm³ size tissue fragments, washed with phosphate buffered saline (PBS, containing 100 U/ml penicillin, 100 U/ml streptomycin) for 2 times, added with double volume of 0.25 mg/ml type II collagenase (Worthington, Freehold, NJ, USA) and placed in shakers to digest at 37 °C for 4, 5 and 7 hours and later filtered through a sterile nylon mesh followed by centrifugation, the precipitated cells were washed with PBS for two times and counted, Trypan blue stained to detect the vitality of tendon fibroblasts, the cells were seeded at the density of 1×10⁶/dish (100 mm culture dish), and cultured in DMEM (Gibco, Gland, Island, NY, USA, containing 10% fetal bovine serum, 300 ug/ml L-glutamine, 50 ug/ml vitamin C, 100 U/ml penicillin, 100 U/ml streptomycin), tendon cells are passaged to the next generation, expanded and digested with 0.25% trypsin to collect the cells before use in the experiment (Figure 2).

### Example 3

The isolation and culture of adipose derived stromal/stem cells

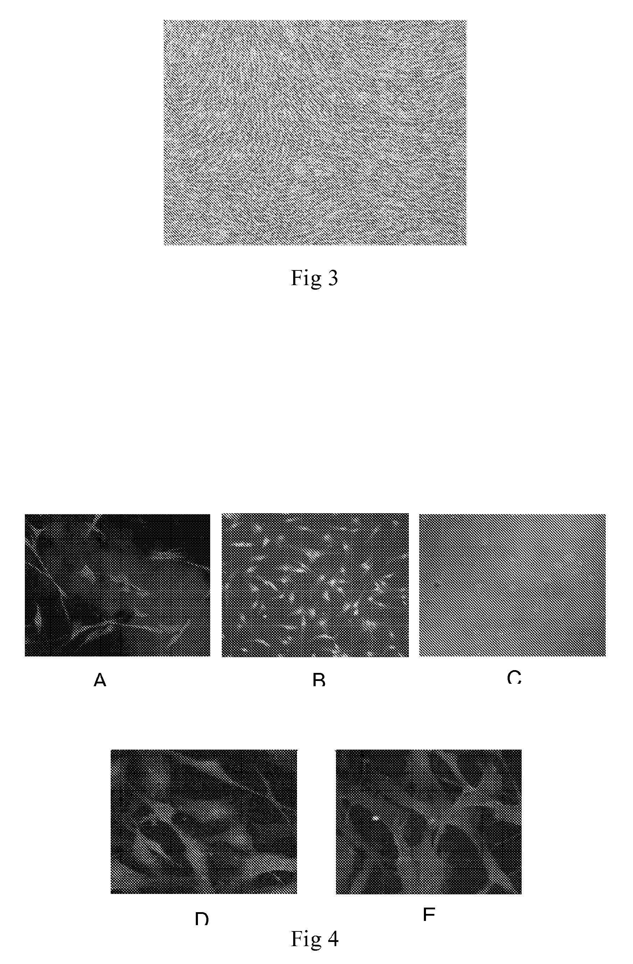

The fresh abandoned human fat tissue is obtained from liposuction under sterile condition, transferred into a culture bottle, washed with saline several times, added with the same volume of 0.075% type I collagenase (Worthington, Freehold, NJ, USA) and placed in shakers to digest for 1h at 37 °C followed by300 g centrifugation for 10 minutes to obtain high density cell pellet, discarding the supernatant and floating adipose tissue, agitating thoroughly and counted, then the cells were counted and cultured at the density of 1×10⁶/dish (100 mm culture dish) under 37 °C, 5% CO₂ and 100% humidity in DMEM (Gibco, Gland, Island, NY, USA, containing 10% fetal bovine serum). The first medium change time is 24 hours post-seeding, the culture dishes were washed with PBS thoroughly to remove the floating cells, adding fresh medium and passaged until substantially confluent (Figure 3). The cells were passaged according to the amount needed and then collected by digestion with 0.25% trypsin for the experiment.

The characterization of adipose-derived stromal/stem cells

The primary adipose-derived stromal/stem cells were cultured on cover slides to 70% confluency, fixed with 4% paraformaldehyde for 15 minutes and added with various antibodies that recognize cell surface antigens followed by the incubation at 37 □for 1h. After washes with PBS, FITC-conjugated secondary antibody (DAKO, Carpinteria, U.S.A) was added and incubated at 37 □ for 30 minutes, washed again with PBS and subject to nuclear counterstain with Heochest 33258 (Sigma, the United States) or Propidium Iodide (Sigma, the United States), and finally the stained cells were observed for the expression of cell surface antigen with Laser Confocal Microscope.

The multiple-lineage differentiation potential of adipose-derived stromal/stem cells

### Chondrogenic differentiation

The 3rd passaged cells were cultured in a micropellet fashion and subject to chondrogenenic induction in DMEM culture medium plus inducing factors at the final concentration of 10 ng/ml TGF-β1(R&D, the United States), 100 ng/ml IGF(R&D, the United States), 0.1µmol/ml Dexamethsone (Sigma, the United States), 6.25µg/ml transferrin (Sigma, the United States).

### Adipogenic induction

The 3rd passage cells were subject to adipogenic induction at day 7 of culture. The induction medium consists of DMEM culture medium plus 0.5mmol/L IBMX (Sigma, the United States), 1µmol/L Dex, 10µmol/L insulin (Sigma, the United States), 200µmol/L indomethacin (Sigma, the United States). The differentiated cells were observed for their morphological changes under inverted phase contrast microscope, and detected at Day 14 of induction.

### Osteogenic induction

The 3rd passage cells were subject to osteogenic induction at day 7 of culture. The induction medium consists of DMEM culture medium plus 2.16g/L β-glycerol phosphate, 10nmol/L vitamin D3. Induction usually lasts for 20 days, then the mineralized nodus was subject to special staining to detect osteogenesis.

### Example 4

The preparation of biomaterial scaffolds

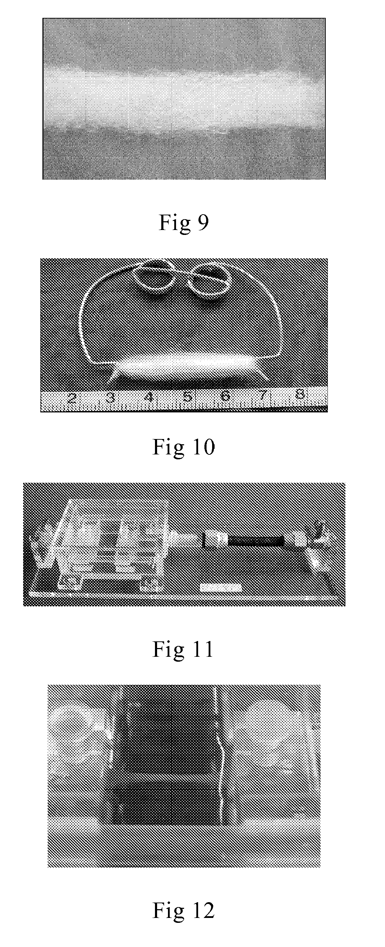

The material fibers were pre-moulded as a bundle form (Figure 9) and mounted on an U-shape spring to apply a constant static tension (Figure 10), soaked in 75% ether for 1 hour for disinfection, then washed with PBS for 3-5 times, disinfect by ultraviolet lamp, air dried for next use.

### Example 5

The vitro construction of tissue engineered tendons

Skin fibroblasts and/or adipose-derived stromal/stem cells were passaged to 2-4 generations, digested with 0.25% trypsin to collect the cells and make a cell suspension with concentration of 2.0×10⁷/ml. The cells in suspension were seeded on pre-moulded PGA fiber scaffold in a culture dish, then the cell-material construct was placed in a culture incubator for 4 hours. Then about 30ml of DMEM culture medium containing 10% fetal bovine serum was added to the culture dish for continued culture in the incubator. Afterwards, medium was replaced every other days to ensure cell nourishment. After about 2 weeks, the cell can secrete enough extracellular matrix, then the construct can be transferred to the cell culture chamber of a tendon bioreactor for culture under dynamic mechanical stretch. The tissue engineered tendon graft was formed after a certain time period (preferably 3∼10 weeks) and can be used for repairing a tendon defect. The tension applied on the seed cell-biomaterial construct in the bioreactor is about 2-20 Newton, with the frequency of about 1-12 hours/day, each for 2-30 seconds, and interval for 5-60 seconds, the traction shift is 5-30% of the length of the graft.

Mentioned in the invention all references in this application are cited as a reference, the same situation as that each reference is singly cited. In addition it should be comprehended that after reading the above-mentioned tuition contents of the invention, the skill area technical personnel can make various change or modifications of the invention. However all related changes or modification of the invention also fall into the scope that are protected by the right of the patent of this application.

## Claims

1. A tissue engineered tendon graft, comprising:
(a) pharmaceutically-acceptable biodegradable material; and
(b) seed cell, which can be inoculated on the described biodegradable material and is selected from: (i) fibroblasts; (ii) ASCs or (iii) mixture of fibroblasts and ASCs according to the ratio of 1:10000-10000:1.

2. The graft of claim 1, wherein, the max tension is 10-80 N.

3. The graft of claim 1, wherein, said seed cells are fibroblast or the mixture of fibroblast and ASCs.

4. The graft of claim 1, wherein, the content of said seed cells is from 1×10⁵ cells/ml to 5×10⁸ cells/ml.

5. The graft of claim 1, wherein, said biodegradable material is in a funicular or a bundle shape.

6. The graft of claim 1, wherein, said fibroblasts and ASCs are autologous or allogeneic origins.

7. The graft of claim 1, wherein, said pharmaceutically-acceptable biodegradable material is selected from the group consisting of polylactic acid, polyglycolic acid, polyhydroxybutyric acid, polyanhydride, polyphosphazene, polyamino acid, pesudo-polyamino acid, polyorthoester, polyesterurethane, polycarbonate, polyethylene glycol, polyethylene oxide, poly-dioxanone, poly (1,4-dioxan-2-one), collagen, gelatin, glycosaminoglycan, chitosan, chitin, alginate, calcium alginate gel, acellular matrix, and their mixtures.

8. A method of preparing the tissue engineered tendon graft of claim 1, comprising the steps of:
mixing the seed cells with the pharmaceutically-acceptable biodegradable material, obtaining a seed cell-scaffold construct, wherein the seed cells are inoculated on the biodegradable material and are selected from:(i) fibroblasts; (ii) ASCs, or (iii) mixture of dermal fibroblasts and ASCs according to the ratio of 1:10000-10000:1.

9. The method of claim 8, further comprising the steps of:
culturing the seed cell-scaffold construct in a bioreactor to obtain the tissue engineered tendon grafts of claim 1.

10. The use of the tissue engineered tendon grafts of claim 1 in the manufacture of a graft for repairing the tendon defect.
